# EUROPEAN PATENT SPECIFICATION

(11) **EP 2 177 238 B1**
(45) Date of publication and mention of the grant of the patent: **09.11.2016**
(21) Application number: 08166578.8
(22) Date of filing: 14.10.2008
(51) Int. Cl.: A61L 27/34, A61L 27/54, A61L 29/08, A61L 29/16, A61L 31/10, A61L 31/16

(54) **Medical device with controllably releasable antibacterial agent**
Medizinische Vorrichtung mit kontrolliert freigesetztem antibakteriellem Wirkstoff
Dispositif médical doté d'un agent antibactérien dont la libération peut être contrôlée

(43) Date of publication of application: 21.04.2010
(73) Proprietor: Dentsply IH AB, 431 21 Mölndal (SE)
(72) Inventor: Nyman, Martin, 428 36, KÅLLERED (SE); Nordholm, Agneta, 433 51, ÖJERSJÖ (SE); Westman, Eva-Helena, 117 38, STOCKHOLM (SE)
(74) Representative: Awapatent AB

(56) References cited:
- EP-A- 0 093 093
- EP-A- 1 688 470
- WO-A-01/53414
- WO-A-98/58989
- WO-A-02/058756
- WO-A-2006/002628

## Description

### Field of the invention

The present invention relates to a medical device comprising a substrate material and a hydrophilic surface coating arranged on at least a part of the surface of said substrate material. The medical device further comprises an antibacterial agent, and provides a predetermined and controllable release of said antibacterial agent. Further, the invention relates to a method for the production of such a medical device.

### Background of the invention

It is known to coat medical devices, e.g. catheters for introduction into human cavities such as blood vessels, digestive organs and the urinary system, with a hydrophilic coating, at least on the surface of the insertable part which is introduced or comes into contact with mucous membranes, etc., during introduction of the device. An advantage with such a hydrophilic coating is that it becomes extremely slippery when it is swelled with water, preferably immediately before introduction into the human body and thus ensures a substantially painless introduction with a minimum of damage on tissue.

A large number of methods are known for the production of hydrophilic surface coatings. A known hydrophilic coating process is e.g. disclosed in EP 0 093 093, where isocyanate is used to form a polyurea network for connecting hydrophilic PVP to the substrate. Further, EP 0 217 771 describes a method of adding an osmolality increasing compound to such a hydrophilic coating in order to improve the water retention properties and low friction of the coating. Further, WO 98/58989 discloses a hydrophilic coating which is cross-linked by means of irradiation, and incorporating a water soluble osmolality increasing compound therein.

However, despite adherence to sterile guidelines etc, the use of medical devices, and in particular introduction of medical devices into natural and artificial body openings, implies a risk of bacterial contamination. For example, insertion and maintenance of urinary catheters poses a problem in relation to catheter-associated infections. When medical devices such as a catheter is introduced into the human cavity, the normal human defense barrier may be penetrated, which can result in introduction of bacteria, fungi, vira, or tissue-like or multiple organized cells. Urinary tract infection (UTI), for instance, is a problem associated with the use of urinary catheters, including hydrophilic catheters with hydrophilic coatings for intermittent use. It is estimated that almost one-quarter of hospitalized spinal cord-injured patients develop symptomatic UTI during their hospital course. Gram-negative bacilli account for almost 60-70%, enterococci for about 25% and Candida species for about 10% of cases of UTI. It is well known that persons practicing intermittent urethral catheterization as a daily routine often have problems with symptomatic UTI.

To this end, and in order to maintain sterility and cleanness of the medical device medical devices, such as urinary catheters, may be coated with an antimicrobial compound for prevention of bacterial infection. US 2006/0240069, for instance, discloses a use of at least one salt of organic acid(s), and preferably a benzoate or a sorbate, as an antimicrobial agent. Further, WO 00/09173 discloses a stabilized composition having antibacterial, antiviral and/or antifungal activity characterized in that it comprises a silver compound. Light stabilized silver composition can be introduced into catheters or similar medical devices.

For many years silver and silver salts have been used as antimicrobial agents. Silver salts, colloids, and complexes have also been used to prevent and to control infection. For example, colloidal metallic silver has been used topically for conjunctivitis, urethritis, and vaginitis. Other metals, such as gold, zinc, copper, and cerium, have also been found to possess antimicrobial properties, both alone and in combination with silver. These and other metals have been shown to provide antimicrobial behavior even in minute quantities, a property referred to as "oligodynamic."

Other examples of medical devices having a hydrophilic coating, and an antimicrobial composition such as silver arranged as a separate layer or introduced into the hydrophilic layer, are disclosed in US 7 378 156 and in EP 1 688 470. WO0153414 discloses antimicrobial agents control released medical devices comprising a substrate material, an antimicrobial silver layer coating and a hydrophilic coating coated over the silver layer.

30 However, a problem with known methods of using oligodynamic metals as antimicrobial and antibacterial agent in medical devices is that it is difficult to control the release of the oligodynamic metal ions. If the release rate is too low, the antibacterial properties would be inadequate, and at the same time a too high release rate may be uncomfortable and even harmful for the patient, and also results in a more costly product. Accordingly, there is a need for an improved medical device of the above-discussed type, where the release rate of the oligodynamic metal ions can be controlled more accurately.

### Summary of the invention

It is therefore an object of the present invention to provide a medical device and a method for producing such a medical device, enabling an improved control of the release rate of an antimicrobial or antibacterial agent, and thereby alleviating the above-discussed problems of the previously known solutions.

This object is achieved with a medical device and a method according to the appended claims.

According to a first aspect of the invention there is provided a medical device according to claim 1.

It has surprisingly been found by the present inventors that by the arrangement of the antibacterial layer beneath the hydrophilic surface coating, the release of the oligodynamic metal ions becomes much more predictable and controllable. Hereby, it becomes possible to effectively tailor the antibacterial properties of the medical device for the intended use, and optimize the antibacterial effect and at the same time effectively preventing any excessive release of antibacterial ions, which may be harmful for the patient.

The hydrophilic surface coating has a thickness when dry that exceeds 5 μm, and most preferably exceeding 10 μm and it is in the range 5-30 μm, and preferably in the range 10-20 μm.

By "oligodynamic metals" are in this application meant any metal that has antimicrobial or antibacterial behavior even in minute quantities. Examples of such oligodynamic metals are silver, e.g. in the form of silver salts, colloids, and complexes, and other metals, such as gold, zinc, copper, and cerium.

The oligodynamic metal of the antibacterial layer preferably comprises silver. Silver ions has a well-documented and effective antibacterial effect, and have also been found to be adequately controllable by means of an upper hydrophilic layer of a suitable thickness.

It is also preferred that the antibacterial layer also comprises a stabilizing agent, such as gold and/or platinum metals.

The hydrophilic coating and the antibacterial layer are advantageously adapted to provide a controlled release of free ions of the oligodynamic metal in the range 0.01 - 1.0 microgram / cm², and preferably in the range 0.05 - 0.5 microgram / cm², and most preferably in the range 0.10 - 0.30 microgram / cm2.

The hydrophilic polymer of the hydrophilic surface coating is preferably at least one of: polyvinyl compounds, polylactames, in particular such as polyvinyl pyrrolidones, polysaccharides, in particular heparin, dextran, xanthan gum, derivatised polysaccharides, hydroxy propyl cellulose, methyl cellulose, polyurethanes, polyacrylates, polyhydroxyacrylates, polymethacrylates, polyacrylamides, polyalkylene oxides, in particular polyethylene oxides, polyvinyl alcohols, polyamides, polyacrylic acid, copolymers of the previously mentioned polymers, copolymers of vinyl compounds and acrylates or anhydrides, copolymers of vinylpyrrolidone and hydroxy ethylmethyl acrylate, cationic copolymers of polyvinyl pyrrolidone and copolymer of polymethylvinyl ether and maleinic acid anyhydride. Most preferably, the hydrophilic polymer is polyvinylpyrrolidone. Preferably the hydrophilic surface coating comprises a hydrophilic polymer which obtains a significantly lowered surface friction when wetted with a wetting liquid. Most preferably, the hydrophilic surface coating comprises polyvinylpyrrolidone (PVP).

The hydrophilic coating preferably forms a polyurea network, whereby said polyurea network forms a covalent bond to said active hydrogen groups in the substrate. Alternatively, the hydrophilic coating may form an ester bond or an epoxy bond to active hydrogen groups in the substrate.

According to one embodiment, coating of the substrate material may be made by a process comprising the steps of: applying sequentially to the surface of the substrate first a solution comprising between 0.05 to 40% (weight to volume) of an isocyanate compound and thereafter a solution containing between 0.5 and 50% (weight to volume) of polyvinylpyrrolidone and curing at an elevated temperature.

However, other hydrophilic coatings are also feasible, such as a coating comprising hydrophilic polymers cross-linked directly to the substrate. The crosslinking may be effected by means of irradiation, e.g. by electron beams or UV light.

The production method according to the present invention is particularly suitable for the production of catheters, and specifically urinary catheters. However, the production method is also useful for many other types of medical devices having a hydrophilic coating. Accordingly, the method according to the present invention is not limited to urinary catheters. Examples of such other medical devices for which the present invention is useful are vascular catheters and other types of catheters, endo and laryngoscopes, tubes for feeding, or drainage or endotracheal use, condoms, wound dressings, contact lenses, implantates, extracorporeal blood conduits, membranes e.g. for dialysis, blood filters and devices for circulatory assistance.

The present invention is useable for a large variety of different substrate materials. However, preferably the substrate is made of a polymer material. The substrate may e.g. comprise at least one of: polyurethanes, latex rubbers, silicon rubbers, other rubbers, polyvinylchloride (PVC), other vinyl polymers, polyesters, polyacrylates, polyamides, polyolefines, thermoplastic elastomers, styrene block copolymers (SBS), or polyether block amid (PEBA).

The coating solution may further comprise a dissolved osmolality increasing compound, such as sodium chloride. Other osmolality increasing compounds, such as urea and the omsolality increasing compounds discussed in EP 0 217 771 are also feasible. .

According to another aspect of the invention there is provided a method of obtaining a controllable release of oligodynamic metal ions from an antibacterial layer comprising oligodynamic metal, comprising the steps of the method of claim 10.

By means of the latter aspect of the invention, similar advantages and specific embodiments as discussed in respect of the first discussed embodiment are obtainable.

These and other aspects of the invention will be apparent from and elicidated with reference to the embodiments described hereinafter.

### Description of preferred embodiments

In the following detailed description preferred embodiments of the invention will be described. However, it is to be understood that features of the different embodiments are exchangeable between the embodiments and may be combined in different ways, unless anything else is specifically indicated. The hydrophilic medical devices may be used for many different purposes, and for insertion into various types of body-cavities. However, the following discussion is in particular concerned with the preferred field of use, urinary catheters, even though the invention is not limited to this particular type of catheters or even this particular type of medical device. It is to be appreciated by those skilled in the art that the inventive concept is not limited to any certain type of devices, but could be used different types of medical devices.

In case of catheters, at least a part of an elongate tube forms an insertable length to be inserted through a body opening of the user, such as the urethra in case of a urinary catheter. By insertable length is normally, in the context of a hydrophilic catheter, meant that length of the elongate tube which is coated with a hydrophilic material, for example PVP, and which is insertable into the urethra of the patient. Typically, this will be 80-140 mm for a female patient and 200-350 mm for a male patient.

The elongate shaft/tube of the catheter is made of a substrate material. The substrates may be made from any polymer material, which are well-known in the technical field and to which the said hydrophilic polymers adhere, such as polyurethanes, latex rubbers, other rubbers, polyvinylchloride, other vinyl polymers, polyesters and polyacrylates. However, preferably the substrate is made of a polymer blend comprising a polyolefin and a composition having molecules with active hydrogen groups, and preferably a composition having molecules with active hydrogen groups. The polyolefin can comprise at least one polymer selected from the group: polyethene, polypropene, and styrene block copolymer (SBS). The composition having molecules with active hydrogen groups can be a polymer having active hydrogen groups bound to the polymer via nitrogen, such as polyamide or polyurethane.

On at least part of the substrate, there is provided an antibacterial coating layer. The antibacterial layer comprises an oligodynamical metal. Many various types of such antibacterial layers are per se known, and may be used for realization of the present invention.

For example, one conventional approach for obtaining antimicrobial medical devices is the deposition of metallic silver directly onto the surface of the substrate, for example, by vapor coating, sputter coating, or ion beam coating.

Another method of coating silver onto a substrate involves deposition or electrodeposition of silver from solution. In order to improve adhesion, inclusion of deposition agents and stabilizing agents, such as gold and platinum metals, may be used, or alternatively it is possible to form chemical complexes between a silver compound and the substrate surface. For example, it is possible to use the antibacterial coatings with silver as described in US 5 395 651, US 5 747 178 and 5 320 908 to Sodervall et al.

A hydrophilic coating is arranged on at least part of the substrate forming the catheter shaft, and on top of the above-discussed antibacterial coating layer the hydrophilic polymer coating may comprise material selected from polyvinyl compounds, polysaccharides, polyurethanes, polyacrylates or copolymers of vinyl compounds and acrylates or anhydrides, especially polyethyleneoxide, polyvinylpyrrolidone, heparin, dextran, xanthan gum, polyvinyl alcohol, hydroxy propyl cellulose, methyl cellulose, copolymer of vinylpyrrolidone and hydroxy ethylmethyl acrylate or copolymer of polymethylvinyl ether and maleinic acid anyhydride. The preferred hydrophilic polymer is polyvinylpyrrolidone.

The hydrophilic surface coating is provided to have a thickness large enough to provide a controlled release of oligodynamic metal ions through the hydrophilic surface coating. The hydrophilic surface coating has a thickness when dry in the range 5-30 μm, and preferably in the range 10-20 μm.

The coating may also comprise an osmolality-increasing compound, as is e.g. taught in EP 0 217 771.

A preferred method for coating of the substrate will now be disclosed in more detail. The outer surface of the elongate shaft is preferably coated with a stable hydrophilic coating by applying sequentially to the surface of the substrate first a solution comprising between 0.05 to 40% (weight to volume) of an isocyanate compound and thereafter a solution containing between 0.5 and 50% (weight to volume) of polyvinylpyrrolidone and curing at an elevated temperature. The isocyanate solution may advantageously contain between 0.5 to 10% (weight to volume) of the isocyanate compound, and may preferably contain between 1 to 6% (weight to volume) of the isocyanate compound. Generally, the isocyanate solution only needs to be in contact with the surface briefly, for example 5 to 60 sec.

Application of the isocyanate solution to the substrate surface results in a coating having unreacted isocyanate groups being formed on the substrate surface. Application of the polyvinylpyrrolidone solution to the substrate surface then results in a hydrophilic polyvinylpyrrolidone-polyurea interpolymer coating being formed. Curing of this hydrophilic coating binds the isocyanate compounds together to form a stable non-reactive network that binds the hydrophilic polyvinylpyrrolidone. To advantage, curing takes place in the presence of a water-containing gas, for example ambient air, to enable the isocyanate groups to react with the water to yield an amine which rapidly reacts with other isocyanate groups to form a urea cross-link. Further, the method may comprise the steps of evaporating the solvent of the isocyanate solution prior to application of the polyvinylpyrrolidone solution and evaporating the solvent of the polyvinylpyrrolidone solution prior to curing of the hydrophilic coating. This may for example be done by air drying.

The isocyanate compound preferably comprises at least two unreacted isocyanate groups per molecule. The isocyanate may be selected from 2,4-toluene diisocyanate and 4,4'-diphenylmethane diisocyanate, or a pentamer of hexamethylene diisocyanate and toluene diisocyanate of cyanurate type, or trimerized hexamethylene diisocyanate biuret or mixtures thereof.

The solvent for the isocyanate compound is preferably one which does not react with isocyanate groups. The preferred solvent is methylene chloride but it is also possible to use ethyl acetate, acetone, chloroform, methyl ethyl ketone and ethylene dichloride, for example.

In order to shorten the necessary reaction times and curing times suitable catalysts for isocyanate curing may be added. These catalysts may be dissolved in either the isocyanate solution or the polyvinylpyrrolidone solution but are preferably dissolved in the latter. Different types of amines are especially useful, for example diamines, but also for example triethylenediamine. Preferably, an aliphatic amine is employed which is volatisable at the drying and curing temperatures used for the coating, and which furthermore is non-toxic. Examples of suitable amines are N,N' diethylethylendiamine, hexamethylendiamine, ethylendiarnine, paradiaminobenzene, 1,3-propandiol-para-aminobenzoic acid diester and diaminobicyclo-octane.

The polyvinylpyrrolidone used preferably has a mean molecular weight of between 104 to 107 with the most preferred mean molecular weight being about 105. Polyvinylpyrrolidone having such a molecular weight is commercially available, for example under the trademark Kollidon® (BASF). Examples of suitable solvents for polyvinylpyrrolidone that may be used are methylene chloride (preferred), ethyl acetate, acetone, chloroform, methyl ethyl ketone and ethylene dichloride. The proportion of polyvinylpyrrolidone in the solution is preferably between 0.5 to 10% (weight to volume) and most preferred between 2 to 8% (weight to volume). The polyvinylpyrrolidone in the solvent is applied by dipping, spraying or the like for a short period of time, e.g. during 5 to 50 sec.

Curing of the coating is preferably performed at a temperature of 50 to 130 deg. C., in for example an oven, for a duration of between 5 to 300 min.

However, it is also feasible to use other types of hydrophilic coatings, such as coatings cross-linked by means of UV or e-beam radiation.

In a preferred embodiment the hydrophilic coating contains an osmolality-increasing compound, for instance an inorganic salt selected from sodium and potassium chlorides, iodides, citrates and benzoates. The osmolality-increasing compound may be applied in the manner detailed in EP 0 217 771 by the same applicant.

### Experimental results

For the experiments, catheters were prepared using a substrate material of a combination of the materials Polypropene, Polyethen Polyamide and Styren-ethen/buten-styren co-polymer, generally sold under the trade name Meliflex.

All the catheter substrates were coated with an antibacterial coating. The coating was applied essentially as disclosed in US 5 395 651 and US 5 747 178. Accordingly, the substrates were first pre-treated with a chromic acid, and then activated by dipping the substrates in a dilute activation solution containing 0.01 - 0.2 grams per liter of a salt containing tin ions, dissolved in acidified demineralized water. After this treatment the substrates were rinsed in demineralized water. Thereafter, the substrates were dipped in a deposition solution comprising silver-containing salt, and more specifically silver nitrate, in an effective amount of no more than 0.10 grams per liter, a reduction agent and a deposition control agent. After deposition, the coated substrates were removed from the deposition solution and rinsed in demineralized water. Finally, the substrates were dipped in a stabilization solution comprising 0.001 - 0.1 grams per liter of salts of platinum and gold in a dilute acid. After stabilization treatment, the substrates were again rinsed in demineralized water, and subsequently dried.

Different amounts of silver was used in the coatings of different types of catheters (see below).

On top of the antibacterial coating, a hydrophilic coating was applied on the substrates to form the inventive examples, whereas some reference catheters were kept without hydrophilic coating, as comparative examples.

The substrates prepared in accordance with the invention were coated in accordance with a known coating process in which isocyanate is used to form a polyurea network for binding PVP. More specifically, the coating according to the comparative example was prepared by dipping the substrates in a primer solution comprising a diisocyanate (named Desmodur IL), which is dissolved in methylene chloride to a concentration of 2% (weight/volume), for 15 seconds. The catheters were thereafter dried at ambient temperature for 60 seconds, and are then dipped for 3 seconds in a solution containing 7% (weight/volume) of polyvinylpyrrolidone (PVP K90) dissolved in methylene chloride. The catheters were then allowed to flush off at 35 deg. C for 30 minutes, and then cured for 60 minutes at 80 deg. C, and were finally allowed to cool to room temperature and rinsed in water.

The hydrophilic coatings had a thickness of 10 - 20 μm when dry, and 100-200 μm when wetted.

Based on the above, the following groups of catheters were tested:
A) Catheters with antibacterial silver coating, but without hydrophilic coating.
B) Catheters with antibacterial silver coating with higher silver concentration than in A), and with hydrophilic coating.
C) Catheters with antibacterial silver coating with about the same concentration as in A), and with hydrophilic coating. Notably, the catheters according to A) and C) were essentially identical, apart from catheters A) not having any hydrophilic coating.

For these catheters, the total silver concentration of the surface was measured, and also the amount of released silver when leached in synthetic urine for 15 seconds, 30 seconds and 5 minutes, respectively. At least three different catheters of each group were tested each time.

The result of these measurements are presented in the following Table 1:

**Table 1: Measurement of leached amount of silver from different catheters**

| Cath. Type | Leach time [s] | Leached amount of silver [μg/cm²] | Average degree of release [%] | Standard deviation | RSD% | 95 CI | Average total amount of silver [μg/cm²] |
|---|---|---|---|---|---|---|---|
| A | 15 | 0.01-0.04 | 2.43 | 1.39 | 56.95 | 1.57 | 0.90 |
| A | 30 | 0.05-0.09 | 7.54 | 2.40 | 31.82 | 2.71 | 0.90 |
| A | 300 | 0.15-0.31 | 24.10 | 8.89 | 36.87 | 10.06 | 0.90 |
| B | 15 | 0.04-0.06 | 3.11 | 1.23 | 39.45 | 1.39 | 2.06 |
| B | 30 | 0.09-0.10 | 4.56 | 0.11 | 2.52 | 0.13 | 2.06 |
| B | 300 | 0.36-0.38 | 17.71 | 0.42 | 2.35 | 0.47 | 2.06 |
| C | 15 | 0.02-0.04 | 3.33 | 1.27 | 38.16 | 1.44 | 0.91 |
| C | 30 | 0.05-0.06 | 5.53 | 0.61 | 11.10 | 0.69 | 0.91 |
| C | 300 | 0.13-0.16 | 16.26 | 1.55 | 9.55 | 1.76 | 0.91 |

The average degree of release of silver ions is calculated as the average quotient of the amount of leached silver and the average total amount of silver.

From these measurement results, the following is particularly notable:
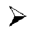 The leached amount of silver varies significantly more for group A, having no hydrophilic coating, than for groups B and C. This effect is further more pronounced for longer leach times.
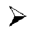 Consequently, the conclusion may be drawn that the hydrophilic coating has a stabilizing effect, making the release of the silver ions from the antibacterial coating much more predictable and controllable.
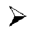 The average amount of leached silver is about the same for group A and C, having approximately the same total amount of silver in the coating.
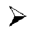 Thus, it may be concluded that the hydrophilic coating has no apparent effect on total level of release of the silver ions from the antibacterial coating.
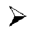 The great improvement in lowering the variation experienced in the release levels of the silver ions is about the same for group B and C. At the same time, the total amount of silver in group B is about doubled compared to group C. Accordingly, this positive effect is apparently not dependent on the total amount of silver ions in the antibacterial coating.

### Conclusion and summary

The invention has now been discussed in relation to different embodiments. However, it should be appreciated by those versed in the art that several further alternatives are possible. For example, many other types of antibacterial coatings comprising oligodynamical metals may be used, as well as other types of hydrophilic coatings. It is further possible to use the invention for other types of catheters than urinary catheters, such as vascular catheters or the like, or for other types of medical devices having a hydrophilic coating.

## Claims

1. A medical device comprising a substrate material, a hydrophilic surface coating arranged on at least a part of the surface of said substrate material, and an antibacterial layer comprising oligodynamic metal arranged between said substrate material and said hydrophilic surface coating, wherein said hydrophilic surface coating has a thickness when dry that exceeds 5 μm but is equal to or less than 30 μm, said thickness thereby being large enough to provide a controlled release of oligodynamic metal ions through the hydrophilic surface coating.

2. The medical device of claim 1, wherein the hydrophilic surface coating has a thickness when dry that exceeds 10 μm

3. The medical device of any one of the preceding claims, wherein the hydrophilic surface coating has a thickness that is equal to or less than 20 μm

4. The medical device of any one of the preceding claims, wherein the oligodynamic metal of the antibacterial layer comprises silver, and preferably also a stabilizing agent, such as gold and/or platinum metals.

5. The medical device of any one of the preceding claims, wherein the hydrophilic surface coating and the antibacterial layer are adapted to provide a controlled release of free ions of the oligodynamic metal in the range 0.01 - 1.0 microgram / cm², and preferably in the range 0.05 - 0.5 microgram / cm², and most preferably in the range 0.10 - 0.30 microgram / cm2.

6. The medical device of any one of the preceding claims, wherein the hydrophilic surface coating comprises a hydrophilic polymer, such as polyvinylpyrrolidone (PVP), which obtains a significantly lowered surface friction when wetted with a wetting liquid.

7. The medical device of any one of the preceding claims, wherein the hydrophilic polymer is at least one of: polyvinyl compounds, polylactames, in particular such as polyvinyl pyrrolidones, polysaccharides, in particular heparin, dextran, xanthan gum, derivatised polysaccharides, hydroxy propyl cellulose, methyl cellulose, polyurethanes, polyacrylates, polyhydroxyacrylates, polymethacrylates, polyacrylamides, polyalkylene oxides, in particular polyethylene oxides, polyvinyl alcohols, polyamides, polyacrylic acid, copolymers of the previously mentioned polymers, copolymers of vinyl compounds and acrylates or anhydrides, copolymers of vinylpyrrolidone and hydroxy ethylmethyl acrylate, cationic copolymers of polyvinyl pyrrolidone and copolymer of polymethylvinyl ether and maleinic acid anyhydride.

8. The medical device of any one of the preceding claims, wherein the medical device is a catheter, and preferably a urinary catheter.

9. The medical device of any one of the preceding claims, wherein the hydrophilic surface coating further comprises an osmolality increasing compound.

10. A method for manufacturing of a medical device with a controllable release of oligodynamic metal ions from an antibacterial layer comprising oligodynamic metal, comprising the steps of:
providing a substrate material;
arranging the antibacterial layer on at least part of the surface of said substrate material; and
arranging a hydrophilic surface coating on top of said antibacterial layer,
wherein said hydrophilic surface coating has a thickness when dry exceeding 5 μm, and being equal to or less than 30 μm, said thickness thereby being large enough to provide a controlled release of oligodynamic metal ions through the hydrophilic surface coating.

11. The method of claim 10, wherein the hydrophilic coating forms a polyurea network, whereby said polyurea network forms a covalent bond to said active hydrogen groups in the substrate.

12. The method of claim 11, wherein the step of coating the substrate material comprises the sub-steps of: applying sequentially to the surface of the substrate first a solution comprising between 0.05 to 40% (weight to volume) of an isocyanate compound and thereafter a solution containing between 0.5 and 50% (weight to volume) of polyvinylpyrrolidone and curing at an elevated temperature.

13. The method of claim 10, wherein the hydrophilic coating is cross-linked to said substrate by means of irradiation.

## Patentansprüche

1. Medizinische Vorrichtung umfassend ein Substratmaterial, eine hydrophile Oberflächenbeschichtung, die auf mindestens einem Teil der Oberfläche des Substratmaterials angeordnet ist, und eine antibakterielle Schicht umfassend oligodynamisches Metall, das zwischen dem Substratmaterial und der hydrophilen Oberflächenbeschichtung angeordnet ist, wobei die hydrophile Oberflächenbeschichtung eine Dicke, wenn sie trocken ist, aufweist, die 5 μm übersteigt, jedoch gleich oder geringer als 30 μm ist, wodurch die Dicke stark genug ist, eine regulierte Freisetzung von oligodynamischen Metallionen durch die hydrophile Oberflächenbeschichtung bereitzustellen.

2. Medizinische Vorrichtung nach Anspruch 1, wobei die hydrophile Oberflächenbeschichtung eine Dicke, wenn sie trocken ist, aufweist, die 10 μm übersteigt.

3. Medizinische Vorrichtung nach einem der vorhergehenden Ansprüche, wobei die hydrophile Oberflächenbeschichtung eine Dicke aufweist, die gleich oder geringer als 20 μm ist.

4. Medizinische Vorrichtung nach einem der vorhergehenden Ansprüche, wobei das oligodynamische Metall der antibakteriellen Schicht Silber und bevorzugt auch ein Stabilisierungsmittel wie Gold und/oder Platinmetalle umfasst.

5. Medizinische Vorrichtung nach einem der vorhergehenden Ansprüche, wobei die hydrophile Oberflächenbeschichtung und die antibakterielle Schicht geeignet sind, eine regulierte Freisetzung freier Ionen des oligodynamischen Metalls im Bereich von 0,01 - 1,0 Mikrogramm/cm² und bevorzugt im Bereich von 0,05 - 0,5 Mikrogramm/cm² und am bevorzugtesten im Bereich von 0,10 - 0,30 Mikrogramm/cm² bereitzustellen.

6. Medizinische Vorrichtung nach einem der vorhergehenden Ansprüche, wobei die hydrophile Oberflächenbeschichtung ein hydrophiles Polymer, wie beispielsweise Polyvinylpyrrolidon (PVP) umfasst, das eine beträchtlich reduzierte Oberflächenreibung bietet, wenn es mit einem Benetzungsmittel benetzt wird.

7. Medizinische Vorrichtung nach einem der vorhergehenden Ansprüche, wobei das hydrophile Polymer mindestens eines ist von:
Polyvinylverbindungen, Polylactamen, insbesondere wie beispielsweise Polyvinylpyrrolidonen, Polysacchariden,
insbesondere Heparin, Dextran, Xanthangummi, derivatisierten Polysacchariden, Hydroxypropylcellulose, Methylcellulose, Polyurethanen, Polyacrylaten, Polyhydroxyacrylaten, Polymethacrylaten, Polyacrylamiden, Polyalkylenoxiden,
insbesondere Polyethylenoxiden, Polyvinylalkoholen, Polyamiden, Polyacrylsäure, Copolymeren der oben erwähnten Polymere,
Copolymeren von Vinylverbindungen und -acrylaten oder - anhydriden, Copolymeren von Vinylpyrrolidon und
Hydroxyethylmethylacrylat, kationischen Copolymeren von Polyvinylpyrrolidon und Copolymer von Polymethylvinylether und Maleinsäureanyhydrid.

8. Medizinische Vorrichtung nach einem der vorhergehenden Ansprüche, wobei die medizinische Vorrichtung einen Katheter und bevorzugt ein Harnkatheter ist.

9. Medizinische Vorrichtung nach einem der vorhergehenden Ansprüche, wobei die hydrophile Oberflächenbeschichtung ferner eine Osmolalität erhöhende Verbindung umfasst.

10. Verfahren zur Herstellung einer medizinischen Vorrichtung mit einer regulierbaren Freisetzung von oligodynamischen Metallionen aus einer antibakteriellen Schicht umfassend oligodynamisches Metall, umfassend die Schritte des:
Bereitstellens eines Substratmaterials;
Anordnens der antibakteriellen Schicht auf mindestens einem Teil der Oberfläche des Substratmaterials; und
Anordnens einer hydrophilen Oberflächenbeschichtung auf der antibakteriellen Schicht, wobei die hydrophile Oberflächenbeschichtung eine Dicke aufweist, wenn sie trocken ist, die 5 μm übersteigt und gleich oder geringer als 30 μm ist, wobei die Dicke dadurch stark genug ist, um eine regulierte Freisetzung von oligodynamischen Metallionen durch die hydrophile Oberflächenbeschichtung bereitzustellen.

11. Verfahren nach Anspruch 10, wobei die hydrophile Beschichtung ein Polyharnstoffnetzwerk bildet, wobei das Polyharnstoffnetzwerk eine kovalente Bindung zu den aktiven Wasserstoffgruppen in dem Substrat bildet.

12. Verfahren nach Anspruch 11, wobei der Schritt des Beschichtens des Substratmaterials die Unterschritte umfasst des: nacheinander Aufbringens auf die Oberfläche des Substrats zuerst einer Lösung umfassend 0,05 bis 40 % (Gewicht zu Volumen) einer Isocyanatverbindung und daraufhin einer Lösung enthaltend 0,5 bis 50 % (Gewicht zu Volumen) Polyvinylpyrrolidon und Aushärtens bei einer erhöhten Temperatur.

13. Verfahren nach Anspruch 10, wobei die hydrophile Beschichtung durch Bestrahlung mit dem Substrat vernetzt wird.

## Revendications

1. Dispositif médical comportant un matériau substrat, un revêtement à surface hydrophile disposé sur au moins une partie de la surface dudit matériau substrat et une couche antibactérienne comportant du métal oligodynamique disposée entre ledit matériau substrat et ledit revêtement de surface hydrophile, ledit revêtement de surface hydrophile ayant une épaisseur à sec qui excède 5 μm mais est inférieure ou égale à 30 μm, ladite épaisseur étant ainsi suffisamment importante pour permettre une libération contrôlée d'ions de métal oligodynamique à travers le revêtement de surface hydrophile.

2. Dispositif médical selon la revendication 1, dans lequel le revêtement de surface hydrophile a une épaisseur à sec qui excède 10 μm.

3. Dispositif médical selon l'une quelconque des revendications précédentes, dans lequel le revêtement de surface hydrophile a une épaisseur qui est inférieure ou égale à 20 μm.

4. Dispositif médical selon l'une quelconque des revendications précédentes, dans lequel le métal oligodynamique de la couche antibactérienne contient de l'argent et de préférence aussi un agent stabilisateur, comme des métaux de type or et/ou platine.

5. Dispositif médical selon l'une quelconque des revendications précédentes, dans lequel le revêtement de surface hydrophile et la couche antibactérienne sont aptes à permettre une libération contrôlée d'ions libres du métal oligodynamique de l'ordre de 0,01 à 1,0 microgramme / cm², et de préférence de l'ordre de 0,05 à 0,5 microgramme / cm², et plus préférentiellement de l'ordre de 0,10 à 0.30 microgramme / cm².

6. Dispositif médical selon l'une quelconque des revendications précédentes, dans lequel le revêtement de surface hydrophile contient un polymère hydrophile, comme du polyvinylpyrrolidone (PVP), ce qui permet d'obtenir une friction de surface nettement réduite lorsqu'il est mouillé avec un liquide de mouillage.

7. Dispositif médical selon l'une quelconque des revendications précédentes, dans lequel le polymère hydrophile est au moins un des éléments suivants : composés de polyvinyle, polylactames, en particulier comme les polyvinyle pyrrolidones, polysaccharides, en particulier l'héparine, la dextrane, la gomme de xanthane, les polysaccharides dérivés, l'hydroxy propyle cellulose, la méthyle cellulose, les polyuréthanes, polyacrylates, polyhydroxyacrylates, polyméthacrylates, polyacrylamides, oxydes de polyalkylène, en particulier oxydes de polyéthylene, alcools de polyvinyle, polyamides, acides polyacryliques, copolymères des polymères mentionnés précédemment, copolymères de composés de vinyle et acrylates ou anhydrides, copolymères de vinylpyrrolidone et hydroxy éthylméthyle acrylate, copolymères cationiques de polyvinyle pyrrolidone et copolymères de polyméthyle vinyle éther and et anyhydride d'acide maléique.

8. Dispositif médical selon l'une quelconque des revendications précédentes, dans lequel le dispositif médical est un cathéter, et de préférence un cathéter urinaire.

9. Dispositif médical selon l'une quelconque des revendications précédentes, dans lequel le revêtement de surface hydrophile comprend en outre un composé augmentant l'osmolarité.

10. Procédé de fabrication d'un dispositif médical à libération contrôlable d'ions de métal oligodynamique à partir d'une couche antibactérienne comprenant du métal oligodynamique, comprenant les étapes de :
prévision d'un matériau substrat ;
disposition de la couche antibactérienne sur au moins une partie de la surface dudit matériau substrat ; et
disposition d'un revêtement de surface hydrophile sur le dessus de ladite couche antibactérienne,
dans lequel ledit revêtement de surface hydrophile a une épaisseur à sec excédant 5 μm et qui est inférieure ou égale à 30 μm, ladite épaisseur étant suffisamment importante pour permettre une libération contrôlée d'ions de métal oligodynamique à travers le revêtement de surface hydrophile.

11. Procédé selon la revendication 10, dans lequel le revêtement hydrophile forme un réseau de polyurée, ledit réseau de polyurée formant une liaison covalente avec lesdits groupes hydrogène actifs dans le substrat.

12. Procédé selon la revendication 11, dans lequel l'étape de revêtement du matériau substrat comprend les sous-étapes de : application séquentielle sur la surface du substrat d'abord d'une solution contenant entre 0,05 et 40 % (ratio poids à volume) d'un composé isocyanate et ensuite d'une solution contenant entre 0,5 et 50 % (ratio poids à volume) de polyvinylpyrrolidone et durcissement à température élevée.

13. Procédé selon la revendication 10, dans lequel le revêtement hydrophile est en liaison croisée avec ledit substrat par irradiation.
